# EUROPÄISCHE PATENTSCHRIFT

(11) **EP 1 784 177 B1**
(45) Veröffentlichungstag und Bekanntmachung des Hinweises auf die Patenterteilung: **29.04.2009**
(21) Anmeldenummer: 05774433.6
(22) Anmeldetag: 12.08.2005
(51) Int. Cl.: A61K 31/405, A61P 29/00, A61P 25/04, A61P 25/24, A61K 31/195, A61K 9/22, A61K 9/24

(54) **FORMULIERUNG FÜR L-TRYPTOPHAN MIT CARBIDOPA/ BENSERAZID**
FORMULATION FOR L-TRYPTOPHANE COMPRISING CARBIDOPA/BENSERAZIDE
FORMULATION POUR L-TRYPTOPHANE COMPRENANT DU CARBIDOPA-BENSERAZIDE

(30) Priorität: 12.08.2004 DE 102004039196
(43) Veröffentlichungstag der Anmeldung: 16.05.2007
(73) Patentinhaber: DR. KAMPRAD KG, 48147 Münster (DE)
(72) Erfinder: KAMPRAD, Joachim, Dr., 48431 Rheine (DE)
(74) Vertreter: Storz, Ulrich
(86) Internationale Anmeldenummer: PCT/DE2005/001428
(87) Internationale Veröffentlichungsnummer: WO 2006/015590

(56) Entgegenhaltungen:
- EP-A- 0 344 158
- WO-A-00/21504
- WO-A-88/04170
- DE-A-6102004 039 19
- FR-A- 2 647 345
- FR-A- 2 654 931
- US-A- 5 188 840
- GENAZZANI A R ET AL: "Effects of L-5HTP with and without carbidopa on plasma beta-endorphin and pain perception: possible implications in migraine prophylaxis." CEPHALALGIA : AN INTERNATIONAL JOURNAL OF HEADACHE. SEP 1986, Bd. 6, Nr. 3, September 1986 (1986-09), Seiten 175-179, XP008060617 ISSN: 0333-1024

## Beschreibung

Die vorliegende Erfindung betrifft die Verwendung von L-Tryptophan und einem peripheren Abbauhemmer von L-Tryptophan zur Herstellung eines Arzneimittels zur Prävention oder Therapie von Schmerzen, Depressionen, Schlafstörungen und anderen Serotoninabhängigen Erkrankungen oder Beschwerden des ZNS, wobei L-Tryptophan in einer retardierten und der periphere Abbauhemmer in einer nicht retardierten Formulierung vorliegen. So daß carbidopa bzw. Benserezid nicht Kontinuierlich im plasma vorhanden ist.

Informationsverarbeitung und -weiterleitung erfolgen im Zentralnervensystem (ZNS) auf dem Boden der neurochemischen Transmission. Die dafür notwendigen chemischen Botenstoffe (Neurotransmitter) werden aus Nahrungsbestandteilen, in der Regel Aminosäuren, synthetisiert und stehen dann den entsprechenden neuronalen Strukturen zur Verfügung. Viele Erkrankungen des Zentralnervensystems beruhen entweder auf dem Mangel eines oder mehrer Neurotransmitter im ZNS oder sind die Folge fehlender oder fehlerhafter Bioverfügbarkeit von Neurotransmittern. Beispiele solcher Botenstoffe sind Serotonin und Dopamin.

Serotonin ist in der Natur weit verbreitet und wird beim Säugetier in relativ hoher Konzentration im Zentralnervensystem (Hypothalamus, Periaquäduktales Grau, Zentrales Höhlengrau, Limbisches System), in der Milz, der Lunge und in den argentaffinen Zellen des Darmtraktes gefunden. Die Konzentration im Vollblut beträgt 0,1-0,3 µg/ml.

Serotonin hat periphere Wirkung auf die glatte Muskulatur der Gefäße des Respirations- und des Gastrointestinaltraktes. Eine besonders bedeutungsvolle Wirkung übt das Serotonin auf das zentrale Nervensystem aus. Hier ist es u.a. an der Schmerzkontrolle, an der Stimmungskontrolle und der Schlafregulation beteiligt.

Dopamin ist ein Catecholamin, das u.a. in Gehirn, Nebenniere und sympathischen Nervenendigungen vorkommt und ist ein Neurotransmitter der hypophyseotropen Hypothalamusgebiete Die Konzentration von Dopamin ist bei Parkinsonismus in den Kernen des extrapyramidal-motorischen Systems vermindert.

Da man Neurotransmitter wie z.B. Serotonin und Dopamin wegen fehlender Bluthirnschrankengängigkeit oder erheblicher Nebenwirkungen dem ZNS nicht direkt zuführen kann, benutzt man gerne biochemische Vorstufen (Präkursoren).

Der Präkursor L-Dopa wird bei der Therapie des Parkinsonismus gerne verabreicht, um einen systemischen Mangel an Dopamin im ZNS, besonders in den sogenannten Stammganglien, auszugleichen. L-Dopa wird allerdings bereits peripher in hohem Maße, d.h. im Blut, sowie im Magen-Darmtrakt aber auch an der Bluthirnschranke (BBB) zu dem im Prinziperwünschten Neurotransmitter abgebaut und erreicht damit nicht oder nur in ungenügender Menge das ZNS. Da Dopamin als solches nicht Blut - Himschrankengängig ist, überschwemmt es die Peripherie, tritt aber praktisch nicht in das Gehirn über. Das hat die bekannten peripheren Nebenwirkungen zur Folge, wie Übelkeit, Erbrechen, Herz-Kreislaufstörungen, Blutdruckveränderungen usw. Um diese Nebeneffekte zu verhindern und die Menge des im ZNS verfügbaren L-Dopa zu steigern, kombiniert man L-Dopa mit peripheren Abbauhemmern, da L-Dopa, wie L-Tryptophan, von der Aminosäuredekarboxylase peripher abgebaut wird. Als Folge davon reichert sich L-Dopa im Plasma an und kann in ausreichend hoher Menge die Blut-Hirnschranke überwinden. Dort wird L-Dopa wie gewünscht zu Dopamin abgebaut. Darüber hinaus wird L-Dopa peripher durch die O-Methyltransferase verstoffwechselt. Der periphere Abbauweg von L-Dopa deckt sich allerdings nur teilweise mit dem peripheren Abbauweg von L-Tryptophan.

Der Präkursor für den Neurotransmitter Serotonin ist L-Tryptophan, dass in den meisten Proteinen zu 1 - 2 % enthalten ist. L-Tryptophan kommt in der natürlichen Nahrung des Menschen vor und ist eine essentielle Aminosäure. Verschiedene Abbauwege des L-Tryptophans sind bekannt. Der Abbau von L-Tryptophan in der Leber über die Tryptophan-2-3-Dioxygenase und über die Kynureninase ist mit über 90% quantitativ am bedeutensten. Hinzu kommt der periphere Abbau von L-Tryptophan über das 5-Hydroxytryptophan (5-HTP) nach Dekarboxylierung zum 5-Hydroxytrytamin (5-HT= Serotonin).

L-Tryptophan wird zur Therapie von Schmerzen mit wechselndem Erfolg verwendet. Darüber hinaus findet die Verwendung von L-Tryptophan alleine bei der Behandlung von Schlafstörungen und Depressionen in Tryptophanhaltigen Fertigarzneimitteln Anwendung. Dabei gibt man den Nahrungsbestandteil L-Tryptophan im Überschuß, um auf diese Art die Bildung der "Antischmerzsubstanz" Serotonin im ZNS zu steigern. Die Versuche L-Tryptophan alleine als wirksames Medikament einzusetzen sind jedoch nicht wirklich geeignet, trotz teilweiser extrem hoher Gaben von L-Tryptophan oder diätetischer Versuche mit Elimination von an der Bluthirnschranke konkurrierenden neutrale Aminosäuren (kompetitive Verdrängung).

Durch den peripheren Abbau des L-Tryptophans außerhalb des ZNS kommt es zur Anreicherung von Serotonin am falschen Ort und damit zu unerwünschten Nebenwirkungen, wie Blutdruckkrisen, chronische Diarrhoe, Bronchospasmus, kardiale Störungen, Magen-Darmstörungen u.a.. Nur eine geringe Menge L-Tryptophan entgeht dem peripheren Abbau und kann ungehindert in des Zentralnervensystem gelangen und dort zum gewünschten Neurotransmitter abgebaut werden. Versuche möglichst große Mengen L-Tryptophan zu geben, um eine wirksame Anreicherung dieser Aminosäure zu erzielen, scheitern an den dann auftretenden Nebenwirkungen und einem gesteigerten intrazerebralen und extrazerebralen Abbau von Serotonin und Tryptophan. Aus diesem Grunde hat diese Aminosäure bisher keine praktische Bedeutung in der Behandlung von z.B. Schmerzen erlangt.

Bekannt ist die Behandlung von Parkinsonpatienten mit L-Dopa-Präparaten in Kombination mit den peripheren Aminosäuredekarboxylasehemmern Benserazid und Carbidopa bzw. Entacapon als 0-Methyltrans-ferasehemmer (COMT-Hemmer). Die Kombination von L-Dopa und dem spezifischen Dekarboxylasehemmer Benserazid nebst einem Hydrocolloid und einigen konventionellen Hilfsstoffen als Präparat mit verzögerter Wirkstofffreisetzung wird in der DE 32 32 873 beschrieben. Nachteilig wirken sich jedoch der relativ schnelle Abbau der Wirkstoffe im Blut aus. Um ein permanentes Angebot von L-Tryptophan an der Bluthirnschranke zu gewährleisten, ist jedoch die Verabreichung der Wirkstoffe entweder in hohen Konzentrationen oder in sehr kurzen Zeitabständen angezeigt. Die hohe Konzentration des peripheren Abbauhemmers weist jedoch erhebliche Nebenwirkungen auf, wohingegen die Verabreichung in kurzen Zeitintervallen oder eine permanente Verabreichung eine stationäre Gabe voraussetzt.

Die Kombination von L-Tryptophan mit einem peripheren Abbauhemmer wie z.B. Benserazid und Carbidopa in einer verzögerten Freisetzungsform zur Behandlung von Schmerzen ist in der EP 0 344 158 B1 beschrieben. Da von Benserazid und Carbidopa der relativ rasche Abbau im Blutplasma (kurze Plasma-Halbwertzeiten) bekannt ist, wird eine retardierte Freisetzung sowohl des L-Tryptophan wie auch des peripheren Abbauhemmers in einer bestimmten Darreichungsform beschrieben. Dabei wird das permanente Angebot von L-Tryptophan an der Blut-Hirnschranke (in retardierter Form) durch ein permanentes Angebot eines peripheren Abbauhemmers (Benserazid oder Carbidopa, ebenfalls in retardierter Form) unterstützt.

Die Verabreichung der Wirkstoffe in retardierter Form bedingt jedoch eine relativ komplexe Formulierung, die in nicht wenigen Fällen mit toxischen Zusatzstoffen einhergeht. Darüber ist es erforderlich die Tablette, Kapsel oder Lösung so zu gestalten, dass eine orale Verabreichung möglich ist. Ferner treten mit dem retardierten peripheren Abbauhemmer erhebliche Nebenwirkungen auf. So leiden die Patienten unter anderem an Tagesmüdigkeit, Übelkeit und Hautreaktionen.

Daher liegt der Erfindung die Aufgabe zu Grunde, ein einfach zu verabreichendes und von Nebenwirkungen weitgehend freies pharmazeutisches Präparat zur Behandlung von Schmerzen, Depressionen, Schlafstörungen oder anderen Serotoninabhängigen Erkrankungen des ZNS zur Verfügung zu stellen.

Die vorliegende Erfindung betrifft die Verwendung von L-Tryptophan und einem peripheren Abbauhemmer von L-Tryptophan zur Herstellung eines Arzneimittels zur Prävention oder Therapie von Schmerzen, Depressionen, Schlafstörungen oder anderen Serotoninabhängigen Erkrankungen des ZNS, wobei L-Tryptophan in einer retardierten und der periphere Abbauhemmer in einer nicht retardierten Formulierung vorliegen. Der periphere Abbauhemmer kann ein peripherer Aminosäuredekarboxylasehemmer, und/oder ein Kynureninasehemmer und/oder ein Tryptophan-2-3-Dioxygenasehemmer sein. Vorzugsweise ist der periphere Abbauhemmer ein peripherer Aminosäuredekarboxylasehemmer, insbesondere (-)-L-α-Hydrazino-3,4-dihydroxy-α-methylhydrozimtsäure (Carbidopa) oder DL-Serin-2-(2,3,4-trihydroxybenzyl)hydrazid-hydrochlorid (Benserazid).

Die Verabreichung von L-Tryptophan erhöht den zentralen Serotoninstoffwechsel bzw. die Serotoninkonzentration im Zentralen Nervensystem. Eine erhöhte Serotoninkonzentration kann insbesondere bei Schmerzen, Depressionen, Schlafstörungen und anderen Serotoninabhängigen Erkrankungen des ZNS therapeutisch oder präventiv eingesetzt werden. L-Tryptophan (Indolyl-3-alanin) stellt eine physiologische Verbindung (essentielle Aminosäure) dar, die zur Behandlung von z.B. Schlafstörungen, Depressionen, Schmerzen und psychotischen Nebenwirkungen der L-Dopa Therapie des Parkinsonismus eingesetzt wird.

Die Kombination von L-Tryptophan in verzögerter Wirkstofffreisetzung (retardierter Formulierung) mit einem peripheren Abbauhemmer von Tryptophan ist aufgrund des Aufnahmemechanismus durch die Blut-Hirn-Schranke für die Behandlung der genannten Erkrankungen von großer Bedeutung.

Vorzugsweise werden in der vorliegenden Erfindung als periphere Abbauhemmer für L-Tryptophan Aminosäuredekarboxylasehemmer und/oder Kynureninasehemmer und/oder Tryptophan-2-3-Dioxygenasehemmer verwendet. Insbesondere bevorzugt werden (-)-L-α-Hydrazino-3,4-dihydroxy-α-methylhydrozimtsäure (Carbidopa) oder DL-Serin-2-(2,3,4-trihydroxybenzyl)hydrazid-hydrochlorid (Benserazid).

Aminosäuredekarboxylasehemmer wirken durch Inhibierung des Enzyms aromatische Aminosäuredekarboxylase. Die verwendeten peripheren Dekarboxylasehemmer Benserazid und Carbidopa sind darüber hinaus auch Hemmer der Kynureninase und Tryptophan-2-3-Dioxygenase und führen über alle 3 Stoffwechselwege (im Gegensatz zu L-Dopa) zu erhöhten L-Tryptophanwerten im Plasma.

Eine dauerhaft hohe Konzentration von Carbidopa oder Benserazid in Form einer retardierten Wirkstofffreisetzung mit Hemmung aller drei Stoffwechselwege von L-Tryptophan (Decarboxylase, Kynureninase und 2-3-Dioxygenase) ist entgegen ursprünglicher Annahmen auf Grund der vom Erfinder überraschenderweise gefundenen Ergebnisse zur optimalen Ausnutzung der Hemmung des peripheren L-Tryptophanabbaus nicht erforderlich.

Der periphere Abbauhemmer kann gleichzeitig mit, vor oder nach L-Tryptophan verabreicht werden.

Die vorliegende erfindungsgemäße Verwendung von retardiertem L-Tryptophan mit nicht retardiertem peripheren Abbauhemmer hat folgende Vorteile: Das erfindungsgemäße Medikament mit nur einer retardierten Komponente, umgeht die Schwierigkeiten bei der galenischen Entwicklung (final formulation) mit zwei retardierten Komponenten. Demzufolge ist der Entwicklungsaufwand geringer, die Darreichungsform weniger komplex und einfacher. Mögliche toxikologische Probleme, die sich aus den Träger- und Zusatzstoffen der Retardierungszusätze ergeben können, werden umgangen. Die Tablette oder Kapsel kann wesentlich kleiner gefertigt werden, wodurch sie besser oral zu verabreichen ist. Die Wirkstoffe können dem Patienten in Form einer Kapsel, Tablette, Lösung, Inhalat oder einer anderen üblichen Darreichungsform verabreicht werden. Durch die einfachere Galenik ist die Darreichungsform kostengünstiger. Es treten weniger Nebenwirkungen auf, die Darreichungsform ist verträglicher.

### Die nachfolgenden Figuren erläutern die Erfindung.

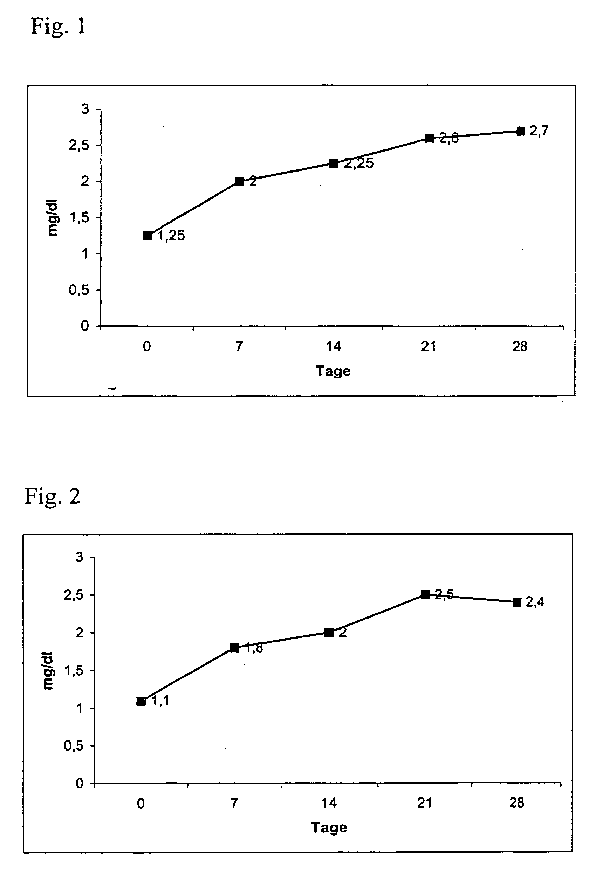

Figur 1 zeigt in einer graphischen Darstellung die Ergebnisse der Verabreichung von L Tryptophan und Benserazid, jeweils eine Retardierung widerspiegelnd, aus einer Studie mit 36 Patienten, wobei nur die Patienten in die Grafik aufgenommen wurden, die das Medikament und nicht ein Plazebo verabreicht bekamen. Der Tryptophanplasmaspiegel ist in mg/dl angegeben.
Figur 2 zeigt in einer graphischen Darstellung die Ergebnisse der Verabreichung von L-Tryptophan und Benserazid, wobei nur die Verabreichung von Tryptophan eine Retardierung widerspiegelt und Benserazid in nicht retardierter Form gegeben wurde, aus einer Studie mit 5 Patienten. Der Tryptophanplasmaspiegel ist in mg/dl angegeben.

### Beispiel 1: Verabreichung von retardiertem L-Tryptophan und retardiertem Benserazid

2 Studien mit insgesamt 58 Schmerzpatienten in 2 Prüfzentren wurden mit retardiertem L-Tryptophan und retardiertem Benserazid durchgeführt. Die Studien waren doppelblind und randomisiert. Eine Studie wurde an 22 Patienten (12 Verum, 10 Plazebo) in der Weserlandklinik in Vlotho, Deutschland, in der Indikation Fibromyalgie durchgeführt. Eine zweite Studie mit 36 Patienten (18 Verum, 18 Plazebo) erfolgte in der Schmerzambulanz des Jakobi-Krankenhauses in Rheine, Deutschland. In der zweiten Studie wurden Patienten mit chronischen Schmerzen behandelt, unabhängig von der dem chronischen Schmerz zugrunde liegenden Erkrankung. Die Wirkstoffe L-Tryptophan und Benserazid wurden häufig und in kleinen Dosen verabreicht, um einen stady state (auch nachts) beider Wirksubstanzen im Plasma zu erreichen. Die Art der Verabreichung spiegelt eine Verabreichung von Wirkstoffen in retardierter Form wider. Die Ergebnisse können daher einer Verabreichung der Wirkstoffe in retardierter Form gleichgesetzt werden. Die Probanden erhielten gleichmäßig über den Tag verteilt um 7, 10, 13, 16 und 19 Uhr, 5 mal 2 Steckkapseln a 200 mg L-Tryptophan / 20 mg Benserazid und um 22 Uhr zwei Retardtabletten a 200 mg L-Tryptophan / 25 mg Benserazid. Die Tryptophanplasmaspiegel wurden kontinuierlich über 4 Wochen kontrolliert. Der L-Tryptophanspiegel im Plasma stieg in beiden Studien kontinuierlich an, erreichte mit 2,6 - 3,4 mg/dl nach 2-3 Wochen sein Maximum und blieb danach konstant.

### Beispiel 2: Verabreichung von retardiertem L-Tryptophan und nicht retardiertem Benserazid

In einer weiteren Studie wurde die Wirkung des Abbauhemmers auf den L-Tryptophanplasmaspiegel überprüft. Dazu wurden 5 Probanden L-Tryptophankonzentrationen verabreicht, die mit den Konzentrationen der ersten Studien (siehe Beispiel 1) identisch waren. Die Versuchspersonen erhielten demzufolge gleichmäßig über den Tag verteilt um 7, 10, 13, 16 und 19 Uhr, 5 mal 2 Steckkapseln a 200 mg L-Tryptophan und um 22 Uhr 2 Retardtabletten a 200 mg L-Tryptophan. Der periphere Abbauhemmer wurde dagegen nur zu drei Zeitpunkten verabreicht, was eine nicht retardierte Verabreichung widerspiegelt. So wurde Benserazid um 7, 15 und 22 Uhr in einer Dosierung von 80 mg pro Einzel-Gabe verabreicht, so dass insgesamt 240. mg Benserazid pro Tag eingenommen wurden (entspricht der Gesamtmenge der beiden ersten Studien). Der Plasmatryptophanspiegel stieg dabei ebenfalls kontinuierlich an, erreichte mit durchschnittlich 2,5 mg/dl sein Maximum nach 3 Wochen und blieb dann bis zum Ablauf der 4. Woche konstant. Es traten im Gegensatz zum Beispiel 1 praktisch keine Nebenwirkungen auf.

### Ergebnis:

Die Studien zeigten, dass neben einer überragenden Schmerzwirkung, die auf L-Tryptophan zurück zu führen war, die kontinuierliche Gabe (entspricht einer Verabreichung in retardierter Form) von L-Tryptophan für eine optimale Wirkung und einen konstant hohen Plasmaspiegel erforderlich ist. Die Studien zeigten darüber hinaus, dass der periphere Abbauhemmer für einen konstant hohen Tryptophanplasmaspiegel und damit für eine optimale Wirkung von L-Tryptophan nicht zwingend kontinuierlich im Plasma vorhanden sein muss. Es war nicht erforderlich, Benserazid ebenfalls in retardierter Formulierung zu verabreichen. Die erzeugten Tryptophanplasmaspiegel waren nach ca. 3 Wochen konstant hoch und korrelierten mit einer überragenden analgetischen Wirkung. Es traten weniger Nebenwirkungen auf, die Verträglichkeit war deutlich besser.

## Patentansprüche

1. Verwendung von L-Tryptophan und (2S)-3-(3,4-Dihydroxyphenyl)-2-hydrazino-2-methylpropionsäure. (Carbidopa) oder DL-Serin-2-(2,3,4-trihydroxybenzyl)hydrazidhydrochlorid (Benserazid) zur Herstellung eines Arzneimittels zur Prävention oder Therapie von Schmerzen, Depressionen und Schlafstörungen; wobei L-Tryptophan in einer retardierten und (2S)-3-(3,4-Dihydroxyphenyl)-2-hydrazino-2-methytpropionsäure (Carbidopa) oder DL-Serin-2-(2,3,4-trihydroxybenzyl)hydrazidhydrochlorid (Benserazid) in einer nicht retardierten Formulierung vorliegen,
so daß Carbidopa bzw. Benserezid nicht kontinuierlich im plasma vorhanden ist.

2. Verwendung nach Anspruch 1, wobei (2S)-3-(3,4-Dihydroxyphenyl)-2-hydrazino-2-methylpropionsäure (Carbidopa) oder DL-Serin-2-(2,3,4-trihydroxybenzyl)hydrazidhydrochlorid (Benserazid) vor, nach oder gleichzeitig mit L-Tryptophan verabreicht wird.

3. Verwendung nach einem der Ansprüche 1-2, wobei das Arzneimittel in Form einer ' Kapsel. Tablette, Lösung oder als Inhalat vorliegt.

## Claims

1. Use of L-tryptophan and (2S)-3-(3,4-Dihydroxyphenyl)-2-hydrazinic-2-methylpropionic acid (Carbidopa) or DL-Serin-2-(2,3,4-trihydroxybenoic)-hydrazide hydrochloride (Benserazide) for the manufacture of a medicament for prevention or therapy of pain, depressions and sleeping disorders,
wherein L-tryptophan is present in a retarded formulation and (2S)-3-(3,4-Dihydroxyphenyl)-2-hydrazinic-2-methyl-propionic acid (Carbidopa) or DL-Serin-2-(2,3,4-trihydroxybenoic)-hydrazide hydrochloride (Benserazide) is present in a non retarded formulation,
in such way that Carbidopa, or Benserazide, respectively, is not present in the plasma in a continuous fashion.

2. Use according to claim 1, wherein (2S)-3-(3,4-Dihydroxyphenyl)-2-hydrazinic-2-methyl-propionic acid (Carbidopa) or DL-Serin-2-(2,3,4-trihydroxybenoic)-hydrazide hydrochloride (Benserazide) is administered prior to, after or simultaneously with L-tryptophan.

3. Use according to claims 1 - 2, wherein the medicament is present in form of a capsule, a tablet, a solution or as an inhalant.

## Revendications

1. Utilisation de L-tryptophane et acide hydrazino-2(dihydroxy-3,4 phenyl)-3 methyl-2 propionique (Carbidopa) ou amino-2 hydroxy-3 propionyl)-1 (trihydroxy-2,3,4 benzyl)-2 hydrazine.chlorhydrate (Benserazide) pour la fabrication d'un médicament pour l'empêchement ou la thérapie de la douleur, les dépressions et les désordres de sommeil, dans lequelle le L-tryptophane est présent dans une fomulation retardée acide hydrazino-2(dihydroxy-3,4 phenyl)-3 methyl-2 propionique (Carbidopa) ou amino-2 hydroxy-3 propionyl)-1(trihydroxy-2,3,4 benzyl)-2 hydrazine.chlorhydrate (Benserazide) est présent dans une formulation non retardée, d'une telle manière qui le carbidopa ou le benserazide ne sont pas présent dans le plasma d'une mode continue.

2. Utilisation selon la revendication 1, dans laquelle acide hydrazino-2(dihydroxy-3,4 phenyl)-3 methyl-2 propionique (Carbidopa) ou amino-2 hydroxy-3 propionyl)-1(trihydroxy-2,3,4 benzyl)-2 hydrazine.chlorhydrate (Benserazide) est administré antérieurement de, après de ou simultanément avec L-tryptophane.

3. Utilisation selon l'une quelconque des revendications 1 - 2, dans laquelle le médicament est présent sous la forme d'une capsule, un comprimé, une solution ou comme inhalant.
